Europäisches Patentamt

European Patent Office

Office européen des brevets

(11) Publication number: **0 579 246 A1**

## EUROPEAN PATENT APPLICATION

(21) Application number: 93111446.6

(22) Date of filing: **16.07.93**

(51) Int. Cl.5: **G01N 33/72**

(30) Priority: **17.07.92 JP 212286/92**

(43) Date of publication of application:
**19.01.94 Bulletin 94/03**

(84) Designated Contracting States:
**DE FR GB IT**

(71) Applicant: **Tosoh Corporation**
**4560, Kaisei-cho**
**Shinnanyo-shi, Yamaguchi-ken(JP)**

(72) Inventor: **Shindo, Yoshiyuki**
**4-3-31-404, Nakanoshima,**
**Tama-ku**
**Kawasaki-shi, Kanagawa-ken(JP)**

(74) Representative: **VOSSIUS & PARTNER**
**Postfach 86 07 67**
**D-81634 München (DE)**

(54) Method for removing unstable type saccharified hemoglobin.

(57) A method of removal of unstable type saccharified hemoglobin is provided which comprises adding, to a sample, one or more organic acids selected from the group of maleic acid, malonic acid, succinic acid, malic acid, phthalic acid, acetic acid, oxalic acid, fumaric acid, tartaric acid, formic acid, lactic acid, and propionic acid, and optionally boric acid. A reagent for use for the above method is also provided. The method is useful for rapid removal of unstable type saccharified hemoglobin in determination of stable type saccharified hemoglobin.

Rank Xerox (UK) Business Services
(3. 10 / 3.6 / 3.3. 1)

The present invention relates to a method for preparing a test sample for determining a stable type saccharified hemoglobin in clinical test.

The saccharified hemoglobin is formed in blood by combination of hemoglobin with sugar in proportion to the blood sugar concentration. The concentration of the saccharified hemoglobin in blood is said to reflect the average blood sugar value of two months ago. Since the saccharified hemoglobin concentration is affected less by physiological conditions in comparison with the blood sugar value and the urine sugar value, it is widely used as the most suitable index for diagnosis of diabetes or observation of the progress of a diabetic patient.

The main constituent of the saccharified hemoglobin is a hemoglobin bonded to glucose at the N-terminal of the $\beta$-chain thereof, and is formed by two-step non-enzymatic reactions: the first step reaction which gives an unstable type saccharified hemoglobin which partially returns reversibly to a free form, and the second step reaction which gives irreversibly the stable type saccharified hemoglobin. To obtain a more precise index of diabetes, it is desirable to measure the stable type saccharified hemoglobin.

The separate analysis of the stable type saccharified hemoglobin is practiced by liquid chromatography or a like method. In the separate analysis, coexisting unstable type saccharified hemoglobin causes an error in the determination result. Accordingly, for the separate analysis, the unstable type saccharified hemoglobin needs to be removed from the analysis sample.

Several methods are known for removal of the unstable type saccharified hemoglobin: a method of standing an erythrocyte-containing blood sample in a physiological saline or a buffer solution containing semicarbazide and aniline; a method of hemolyzing a whole blood sample with a hemolyzing agent containing boron or the like and then standing the sample; and so forth. Such methods require the washing of the erythrocyte several times with the aforementioned solution before the standing, or require standing at 37°C for more than 4 hours, and are troublesome and time-consuming.

Particularly when a sample contains unstable type hemoglobin in a larger amount, the degree of removal thereof is as low as 50 to 60 % even if the sample is left standing with boric acid added thereto at 37°C for 2 hours.

As described above, the unstable type saccharified hemoglobin cannot be readily and rapidly removed by any of known methods.

The present invention intends to provide a method for rapid removal of unstable type of saccharified hemoglobin in determination of stable type saccharified hemoglobin.

The present invention also intends to provide a reagent for removal of unstable type saccharified hemoglobin.

The method of removal of unstable type saccharified hemoglobin of the present invention comprises adding to a sample one or more organic acids selected from the group of maleic acid, malonic acid, succinic acid, malic acid, phthalic acid, acetic acid, oxalic acid, fumaric acid, tartaric acid, formic acid, lactic acid, and propionic acid.

The reagent for removal of unstable type saccharified hemoglobin of the present invention comprises one or more organic acids selected from the group of maleic acid, malonic acid, succinic acid, malic acid, phthalic acid, acetic acid, oxalic acid, fumaric acid, tartaric acid, formic acid, lactic acid, and propionic acid.

The samples to be treated by the method of the present invention include one which contains at least both of stable type saccharified hemoglobin and unstable type saccharified hemoglobin. More specifically, the sample includes whole blood, and suspensions of blood cell components.

The present invention provides a method of removal of unstable type saccharified hemoglobin from the above-mentioned sample by addition of one or more organic acids selected from the group of maleic acid, malonic acid, succinic acid, malic acid, phthalic acid, acetic acid, oxalic acid, fumaric acid, tartaric acid, formic acid, lactic acid, and propionic acid. The concentration of the reagent to be added is not specially limited. However, since the effect of the removal of unstable type saccharified hemoglobin depends on the concentration of the reagent, a higher concentration of the organic acid is preferred. Specifically, the concentration is not lower than 1 mM. An organic acid remaining at a high concentration after the removal of the unstable type saccharified hemoglobin frequently affects adversely the determination accuracy in the subsequent analysis. Therefore, the organic acid concentration is preferably not higher than about 1000 mM. Accordingly, in the present invention, the organic acid is used at a concentration in the range of from about 1 to 1000 mM, preferably from 10 to 200 mM. More preferably the organic acid is used at a concentration of from 50 to 150 mM where a high effect of removal of the unstable type saccharified hemoglobin is achievable and the subsequent treatment is less adversely affected. The organic acid solution is adjusted preferably to be at a pH level of from about 5.5 to 6 in order to prevent denaturation of the stable type saccharified hemoglobin in the sample.

Addition of boric acid together with the organic acid enables more effective removal of the unstable type saccharified hemoglobin. Therefore, the addition of both of an organic acid and boric acid is preferred in the present invention particularly for the sample containing a large amount of unstable type saccharified hemoglobin. The concentration of the boric acid to be added is not limited specially. However, since the higher effect of the removal is achievable at the higher concentration of boric acid, the boric acid is used preferably at a high concentration, specifically at a concentration higher than about 0.1 M. When the removal treatment of the present invention is conducted at a low temperature (e.g., from about 4 to about 10°C), or when the reagent containing the organic acid and boric acid is stored at a low temperature (e.g., about 4 to about 10°C), the boric acid is contained preferably at a concentration where the boric acid does not deposit (not higher than about 0.6 M). Accordingly, the boric acid is preferably used at a concentration in the range of from about 0.1 to about 0.6 M, more preferably from 0.3 to 0.5 M in the present invention. In particular, the concentration of about 0.4 M is preferred since at this level of concentration the unstable type saccharified hemoglobin is effectively removed and the boric acid is less liable to deposit.

With a sample which contains erythrocytes, the organic acid or the combination of the organic acid and boric acid is added at the aforementioned concentrations and in such an amount that the sample is diluted sufficiently to cause hemolyzation of the erythrocytes in the sample. Thereby the unstable type saccharified hemoglobin and the stable type saccharified hemoglobin are liberated from the erythrocytes into the liquid phase. For hemolysis of the erythrocytes, the organic acid or the combination of the organic acid and boric acid is added preferably in an amount to give 50-fold dilution or 100-fold dilution of the sample.

The unstable type saccharified hemoglobin can be removed by adding the organic acid or the combination of the organic acid and boric acid to the sample and leaving the mixture standing at room temperature for about 5 minutes. The sample is preferably heat treated in order to prevent denaturation of stable type of saccharified hemoglobin by the added reagent and to complete the removal of the unstable type saccharified hemoglobin in a short time. This heat treatment may be conducted at a temperature above room temperature and below about 60°C. The heat treatment at about 50°C for 1 to 2 minutes is effective to remove the unstable type saccharified hemoglobin without denaturation of the stable type saccharified hemoglobin.

The unstable type saccharified hemoglobin in the sample is removed by the aforementioned procedures. More correctly, the unstable type saccharified hemoglobin is decomposed into hemoglobin and glucose. Therefore, the treated sample is usable for separate analysis of the stable type saccharified hemoglobin. The stable type saccharified hemoglobin in the sample prepared according to the present invention can be separated and determined by a method of, for example, a commercial mini-column high-speed liquid chromatography, ion-exchange chromatography, or a like method.

As described above, the present invention enables the removal of unstable type saccharified hemoglobin from the sample by addition, to the sample, of one or more organic acids selected from the group of maleic acid, malonic acid, succinic acid, malic acid, phthalic acid, acetic acid, oxalic acid, fumaric acid, tartaric acid, formic acid, lactic acid, and propionic acid, or a combination of the above organic acid and boric acid. Even when a large amount of unstable type saccharified hemoglobin is contained in the sample, the present invention enables rapid selective removal of the unstable type saccharified hemoglobin and enables preparation of a sample for precise determination of stable type saccharified hemoglobin. By addition of the organic acid, 90 % or more of the unstable type saccharified hemoglobin is removed. The resulting sample does not cause disturbance in the chromatogram of the liquid chromatography. Therefore, the present invention is effective for preparation of a sample for precise and separate determination of stable type saccharified hemoglobin.

The present invention is described more specifically by reference to examples.

Example

1. Preparation of Sample:

To fractions of fresh blood containing sodium ethylenediamine-tetraacetate as an anticoagulant, glucose was added at concentrations of 200, 400, 600, 800, and 1000 mg/dL, respectively. The fractions of the fresh blood were left standing at 37°C for 2 hours to increase unstable type saccharified hemoglobin intentionally. These fresh blood fractions were used in the experiments below.

The reagents (a) and (b), and the comparative reagents (c) and (d) were prepared as below.

(a): Reagent containing an organic acid and boric acid (of the present invention):

Solution containing 0.1% triton X-100, 0.1% EDTA-4Na (ethylenediaminetetraacetic acid tetrasodium salt), 0.1% sodium azide, 0.4M boric acid, and 0.05M organic acid (one of maleic acid, malonic acid, acetic acid, succinic acid, malic acid, and phthalic acid) were prepared, and pH of the solutions was adjusted to 5.7 by addition of a sodium hydroxide solution.

(b): Reagent containing an organic acid (of the present invention):

Solution containing 0.1% Triton X-100, 0.1% EDTA-4Na, 0.1% sodium azide, and 0.05M organic acid (one of maleic acid, malonic acid, acetic acid, succinic acid, malic acid, and phthalic acid) were prepared, and pH of the solutions was adjusted to 5.7 by addition of a sodium hydroxide solution.

(c): A solution containing 0.1% Triton X-100, 0.1% EDTA-4Na, and 0.1% sodium azide (pH: about 7.4)

(d): A solution containing 0.1% Triton X-100, 0.1% EDTA-4Na, 0.1% sodium aside (pH: about 6.5), and 0.4M boric acid (pH: about 6.5)

A commercial apparatus (HLC-723 GHb III, made by Tosoh Corporation) was employed for analysis of stable type saccharified hemoglobin. The reagent (a), (b), (c), or (d) was placed in the apparatus, and a blood sample was placed in an automatic sampler. The blood sample was diluted 200-fold with the reagent automatically by means of a dilution device. When the reagent (c) was added, the diluted matter was not heat-treated, but when the reagent (a), (b), or (c) was used, the diluted matter was heat-treated at 50°C for 1.5 minutes.

The sample prepared as above was subjected to separation of stable type saccharified hemoglobin by use of a saccharified hemoglobin separation column (TSK-gel, Glyco HS type, made by Tosoh Corporation), and the separated stable type saccharified hemoglobin was detected by light absorption at wavelength of 415 nm. The stable type saccharified hemoglobin exhibits remarkable light absorption at wavelength of 415 nm. In the separation with the column, the eluent used was the one prepared as eluting solutions for stable type saccharified hemoglobin separation analysis (Eluents for TSK-gel Glyco HS column: First HS Eluent, Second HS Eluent, and Third HS Eluent, made by Tosoh Corporation).

Table 1 shows the results of measurement for the samples prepared by use of the reagents (a), (b), (c), or (d) in terms of the ratio (%) of stable type saccharified hemoglobin to total hemoglobin in the sample. When neither the organic acid nor boric acid was used (Reagent (c)), or when the organic acid was not used (Reagent (d)), the measured values rose depending on the amount of added glucose. Assuming that the concentration of the stable type saccharified hemoglobin in the blood sample is definite, the rise of the measured values is caused by the unstable type saccharified hemoglobin formed by the added glucose. Thus, the unstable type saccharified hemoglobin, when present in a large amount, cannot be removed satisfactorily. On the contrary, when Reagent (a) or (b) of the present invention is used, the unstable type saccharified hemoglobin is removed effectively and the measured value of the stable type saccharified hemoglobin is affected less even when the unstable type saccharified hemoglobin is increased by addition of glucose.

Table 1

EP 0 579 246 A1

|  | Reagent (a) | | | | | |
|---|---|---|---|---|---|---|
|  | Maleic acid | Malonic acid | Acetic acid | Succinic acid | Malic acid | Phthalic acid |
| Fresh blood | 5.95 | 5.90 | 5.90 | 6.20 | 6.10 | 5.90 |
| Glucose-containing fresh blood (200 mg/dL) | 5.98 | - | - | - | - | - |
| Glucose-containing fresh blood (400 mg/dL) | 5.94 | - | - | - | - | - |
| Glucose-containing fresh blood (600 mg/dL) | 6.18 | 6.20 | 6.10 | 6.80 | 6.50 | 6.00 |
| Glucose-containing fresh blood (800 mg/dL) | 6.14 | - | - | - | - | - |
| Glucose-containing fresh blood (1000 mg/dL) | 6.01 | 6.30 | 6.50 | 7.60 | 7.50 | 6.10 |

(continued)

Table 1 (continued)

| | Reagent (b) | | | | | | Reagent (c) | Reagent (d) |
|---|---|---|---|---|---|---|---|---|
| | Maleic acid | Malonic acid | Acetic acid | Succinic acid | Malic acid | Phthalic acid | (c) | (d) |
| Fresh blood | 6.00 | 6.00 | 6.00 | 6.20 | 6.10 | 5.90 | 6.17 | 6.00 |
| Glucose-containing fresh blood (200 mg/dL) | 6.10 | 6.10 | 6.10 | 6.20 | 6.20 | 5.90 | 6.96 | 6.25 |
| Glucose-containing fresh blood (400 mg/dL) | - | - | - | - | - | - | 7.62 | 6.67 |
| Glucose-containing fresh blood (600 mg/dL) | - | - | - | - | - | - | 8.58 | 7.24 |
| Glucose-containing fresh blood (800 mg/dL) | - | - | - | - | - | - | 9.05 | 7.46 |
| Glucose-containing fresh blood (1000 mg/dL) | 6.60 | 6.50 | 6.60 | 7.60 | 7.50 | 6.30 | 9.53 | 7.74 |

**Claims**

1. A method of removal of unstable type saccharified hemoglobin from a sample, comprising adding to the sample one or more organic acids selected from the group of maleic acid, malonic acid, succinic acid, malic acid, phthalic acid, acetic acid, oxalic acid, fumaric acid, tartaric acid, formic acid, lactic acid, and propionic acid.

2. A method of claim 1, wherein boric acid is further added to the sample.

3. A method of claim 1 or claim 2, wherein the sample is heat-treated.

4. A reagent for removal of unstable type saccharified hemoglobin, comprising one or more organic acids selected from the group of maleic acid, malonic acid, succinic acid, malic acid, phthalic acid, acetic acid, oxalic acid, fumaric acid, tartaric acid, formic acid, lactic acid, and propionic acid.

5. A reagent of claim 4, wherein the reagent further comprises boric acid.

| | DOCUMENTS CONSIDERED TO BE RELEVANT | | EP 93111446.6 | |
|---|---|---|---|---|
| Category | Citation of document with indication, where appropriate, of relevant passages | Relevant to claim | CLASSIFICATION OF THE APPLICATION (Int. Cl.5) | |
| X | US - A - 4 879 039 (TAKAHASHI ET AL.) * Column 3, lines 33-42 * | 1-5 | G 01 N 33/72 | |
| X | EP - A - 0 271 996 (SCRIPPS CLINIK AND RESEARCH FOUNDATION) * Abstract; claim 1 * | 1 | | |
| X | US - A - 4 876 188 (SMITH ET AL.) * Column 3, lines 6-30 * | 1 | | |
| A | EP - A - 0 319 454 (SEKISUI KAGAKU KOGYO KABUSHIKI KAISHA) * Abstract; claims * | 1 | | |
| A | EP - A - 0 064 758 (PANCHEM GESELLSCHAFT FÜR CHEMISCHE PRODUKTE GMBH) * Abstract * | 1 | TECHNICAL FIELDS SEARCHED (Int. Cl.5) G 01 N | |

The present search report has been drawn up for all claims

| Place of search | Date of completion of the search | Examiner |
|---|---|---|
| VIENNA | 05-10-1993 | SCHNASS |

EPO FORM 1503 03.82 (P0401)